# EUROPEAN PATENT APPLICATION

(11) **EP 1 935 491 A1**
(43) Date of publication of application: **25.06.2008**
(21) Application number: 07022554.5
(22) Date of filing: 21.11.2007
(51) Int. Cl.: B01L 3/00, G01N 33/548, G01N 33/543

(54) **Microchannel chip comprising a reagent in a heat soluble binder**

(30) Priority: 22.11.2006 JP 2006316150; 15.11.2007 JP 2007296998
(71) Applicant: FUJIFILM Corporation, Minato-ku Tokyo (JP)
(72) Inventor: Kato, Kota, Minami-Ashigara-shi Kanagawa (JP); Iwaki, Yoshihide, Ashigarakami-gun Kanagawa (JP); Wakabayashi, Akira, Minami-Ashigara-shi Kanagawa (JP); Karaki, Hideyuki, Minami-Ashigara-shi Kanagawa (JP)
(74) Representative: HOFFMANN EITLE

(57) **Abstract**

A microchannel chip for introducing a liquid sample, includes: a micro channel; and a reagent that is mixed with a heat soluble binder and is carried at a predetermined position in the micro channel, wherein dissolution of the reagent is promoted at the predetermined position as the temperature of the liquid sample rises from the temperature when the liquid sample is introduced.

## Description

### Background of the Invention

### 1. Field of the Invention

This invention relates, to a microchannel chip for previously carrying a dry reagent in a channel and introducing a liquid reagent, thereby dissolving them.

### 2. Description of the Related Art

In recent years, a method of using a microchannel chip has been proposed as a system for realizing analysis and chemical reaction treatment of a trace sample inexpensively and rapidly.

The microchannel chip is a chip applied to an inspection apparatus for supplying liquid to the chip and executing inspection. For example, a biochemical treatment apparatus disclosed in JP-A-2006-170654, etc., exists as the inspection apparatus; it is provided with a stage for placing a biochemical reaction cartridge (microchannel chip) having chambers (treatment spaces) and a micro channel for allowing the chambers to communicate with each other, move means for moving liquid through a channel, detection means for detecting the presence or absence of liquid in the chamber or the liquid quantity, and determination means for determining the move result of the liquid according to information of the liquid in the chamber detected by the detection means, thereby guiding the sample subjected to preliminary treatment in the micro channel into the chamber and analyzing the sample from chemical reaction or biochemical reaction of the inspection reagent and the sample in the chamber.

The preliminary treatment of a sample in the micro channel is treatment of mixing a reaction promotion substance (reagent) in the sample so that the inspection reagent and the sample held in the chamber react with each other efficiently or treatment of mixing a predetermined reaction substance in the sample to isolate or dissolve and amplify a specific component in the sample, for example.

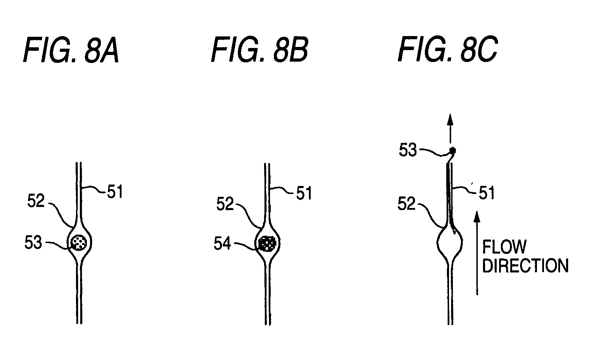

Proposed is a microchannel chip carrying a reagent used for preliminary treatment, reaction treatment for analysis, etc., on a part of an inner wall face of a micro channel with the reagent placed in a dry state previously at the chip manufacturing time so that the preliminary treatment or the reaction treatment for analysis can be conducted easily. (For example, refer to JP-A-2004-194652 and JP-A-2006-133003.) Proposed as a specific method of carrying a dry reagent on an inner wall face of a micro channel in such a microchannel chip is a method of providing a cell (treatment space) 52 at a midpoint in a micro channel 51, for example, as shown in FIG. 8A (in the figure, the channel is widened considering effective mixing) and putting a liquid reagent 53 as a drip on the inner wall face of the cell 52 and then evaporating the moisture of the liquid reagent 53, thereby providing a state in which a reagent 54 in a dry state is fixedly secured to the inner wall face of the cell as shown in FIG. 8B.

In such a microchannel chip, if a sample is allowed to flow into a micro channel, the sample flowing through the micro channel comes in contact with a dry reagent carried on a part of the micro channel and the dry reagent coming in contact with the sample is eluted into the sample, whereby mixing of the reagent and the sample starts and thus the mixing proceeds in the closed space with a small amount of the reagent, so that it is made possible to execute sample analysis work easily.

### Summary of the Invention

However, if a liquid reagent is put as a drip on the inner wall face of the cell 52 and then the moisture in the liquid reagent is evaporated and the reagent 54 is carried as described above, the adhesive strength between the carried reagent 54 and the inner wall face of the cell 52 is low and if a sample is allowed to flow into the micro channel 51, the dry reagent 54 peels off from the inner wall face of the cell 52 like a lump and is made to flow to the outside of the cell 52 as shown in FIG. 8C because of shock, etc., when the sample flows into the cell 52 and starts to come in contact with the carried reagent 54.

In JP-A-2004-194652, the reagent fix position is not widened and it is considered that the liquid flow is in one direction and as with the case shown in FIG. 8C, a dry reagent dissolves rapidly at the normal temperature and flows out in the flow direction immediately when a liquid flow comes. In JP-A-2006-133003, a reagent cell is connected indirectly to a channel and it is feared that dissolving of a reagent will be delayed or a dissolved reagent will not be uniformly mixed in the whole area of a sample, degrading the accuracy of the later reaction treatment and analysis treatment.

If the reagent to be dissolved and mixed in a sample peels off from the inner wall face of the cell and flows, the position where the reagent is actually dissolved and mixed in liquid shifts from a predetermined position in the cell assumed at the beginning. To chemically or biochemically react a sample and a reagent with each other for analysis, heating, etc. , needs to be added to promote the reaction. However, if the reagent is not uniformly mixed, a problem of variations in the reaction, delay of the reaction, or the like also occurs.

It is a common practice to adopt a configuration of executing dissolving treatment, mixing treatment, chemical reaction treatment, biochemical reaction treatment, inspection treatment, etc., of a reagent at different positions on a micro channel so that dissolving or mixing of the reagent is accomplished with predetermined accuracy and chemical reaction treatment or biochemical reaction treatment can be executed for a sample in which the reagent is uniformly mixed if the reagent peels off and flows.

However, if the treatment positions are thus separated, the overall length of the micro channels formed on the chip becomes longer, the channels become a complicated branch structure, and the processing procedure also becomes complicated, leading to problems of upsizing of the microchannel chip and an increase in the manufacturing cost.

It is therefore an object of the invention to provide a microchannel chip wherein a reagent carried at a predetermined position in a micro channel, to be dissolved and mixed in inspected liquid can be reliably dissolved and mixed in the inspected liquid at the initially assumed carry position for improving the accuracy of dissolving treatment and mixing treatment and the later dissolving treatment and mixing treatment can be executed at the initial carry position for reducing the number of the treatment positions on the micro channel, thereby realizing simplifying of the micro channel formed on the chip and shortening of the channel length for miniaturizing the chip and reducing the manufacturing cost.

The solutions of above-mentioned objects can be achieved by the following configurations.
(1) A microchannel chip for introducing an inspected liquid, comprising:
   a micro channel; and
   a reagent that is mixed with a heat soluble binder and is carried at a predetermined position in the micro channel,
   wherein dissolution of the reagent is promoted at the predetermined position as temperature of the inspected liquid rises from temperature when the inspected liquid is introduced.
   According to the configuration, a substance having adhesion is adopted as a heat soluble binder to be mixed with the reagent carried at a predetermined position in the micro channel, whereby the adhesive strength between the reagent and the channel wall face can improved drastically.
   Thus, the reagent is prevented from peeling off from the carry position and flowing as it only comes in contact with the inspected liquid supplied in the micro channel.
   If the temperature after the inspected liquid is introduced into the carry position of the reagent is raised to the stipulated temperature, dissolution of the heat soluble binder coming in contact with the inspected liquid is promoted, and dissolution and mixing of the reagent are also promoted and it is ensured that dissolving treatment and mixing treatment are started and continued at the initial carry position. Thus, for example, the inspected liquid is retained for a given time in a given section with the carry position of the reagent between or the inspected liquid is made to go and return in a given section with the carry position of the reagent between, whereby the reagent can be reliably dissolved and mixed in the inspected liquid and accuracy of the dissolving treatment and the mixing treatment can be improved.
   Further, if the characteristic of a heat soluble binder is previously adjusted so that the dissolution promotion temperature of the heat soluble binder becomes the reaction promotion temperature of the reagent and the inspected liquid, the dissolved and mixed reagent can be made to react with the inspected liquid efficiently, and the later reaction treatment and analysis treatment can be executed on the periphery of the initial carry position. That is, in addition to the dissolving treatment, the mixing treatment, and the amplification treatment, the later reaction treatment and analysis treatment can be executed only at the initial carry position of the reagent or on the periphery of the initial carry position.
   Therefore, the number of the treatment positions on the micro channel can be reduced as compared with the related art case where the dissolving treatment, the mixing treatment, the reaction treatment, and the analysis treatment can be executed at different positions on the micro channel, so that simplifying of the micro channel formed on the chip and shortening of the channel length can also be realized for miniaturizing the chip and reducing the manufacturing cost.
(2) The microchannel chip as described in (1) above, wherein the temperature of the inspected liquid rises to 35°C or more and 60°C or less.
   In so doing, the dissolution promotion start timing of the heat soluble binder and the reagent can be managed by the setting of the rising temperature of the inspected liquid and at the same time, for example, if the reagent is a dry primer used for the purpose of amplification and detection of a target nucleic acid in blood, the reaction temperature of the dry primer can also be maintained for promoting the reaction.
(3) The microchannel chip as described in (1) or (2) above,
   wherein the heat soluble binder is gelatin or hydroxypropylcellulose.
   In so doing, gelatin or hydroxypropylcellulose has heat solubility satisfying (2) mentioned above and at the same time, is also has strong adhesion, so that carrying of the reagent to a predetermined position on the micro channel is facilitated and the configuration described above in (2) can be realized easily.
(4) The microchannel chip as described in any of (1) to (3) above,
   wherein the heat soluble binder is contained in the reagent in a solidified state 20% or more and 98% or less at a weight ratio of the heat soluble binder to the reagent.
   The heat soluble binder contained in the reagent on the micro channel becomes an impurity when it dissolves into inspected liquid. Therefore, it is desirable that the content of the heat soluble binder should be lowered as much as possible. However, to optimally adjust the required time and the reaction speed for uniformly dissolving and mixing the reagent in inspected liquid, it might often be effective to suppress the dissolution speed of the reagent by raising the content of the heat soluble binder. From such a viewpoint, if the content of the heat soluble binder is regulated as mentioned above, solubility of the reagent can be controlled flexibly in response to the composition, the analysis purpose, etc., of the inspected liquid.
(5) The microchannel chip as described in any of (1) to (4) above,
   wherein the reagent causes nucleic acid amplification reaction.
   In so doing, the nucleic acid amplification reaction can be executed efficiently at the carry position of the reagent or on the periphery of the carry position.
(6) The microchannel chip as described in (5) above,
   wherein the nucleic acid amplification reaction is executed at a constant temperature.
   In so doing, the nucleic acid amplification reaction becomes isothermal amplification reaction and the activity of a used enzyme can be maintained constant and thus the nucleic acid amplification reaction stably proceeds, the reliability and the treatment accuracy of the reaction treatment can be improved, and the required time can be shortened.
(7) The microchannel chip as described in any of (1) to (6) above,
   wherein the reagent is a primer of an origin of nucleic acid amplification reaction.

In so doing, it is made possible to control the dissolution promotion start of the reagent at the carry position of the reagent so that it is the start of the nucleic acid amplification reaction, and thus management of the reaction time, etc., is facilitated, whereby the nucleic acid amplification reaction can be well controlled and advanced.

### Brief Description of the Drawings

In the accompanying drawings:
FIG. 1 is an exploded perspective view of one embodiment of a microchannel chip according to the invention;
FIGS. 2A to 2C are schematic representations of the configuration of a channel substrate forming the microchannel chip shown in FIG. 1; FIG. 2A is a plan view of the channel substrate; FIG. 2B is a drawing taken on arrow A in FIG. 2A; and FIG. 2C is a drawing taken on arrow B in FIG. 2B;
FIG. 3A is an enlarged drawing of part C of a micro channel shown in FIG. 2C; FIG. 3B is a schematic representation of a state in which a reagent is put as a drip on an inner wall face of a cell in FIG. 3A; and FIG. 3C is a schematic representation of action when inspected liquid flows into the cell;
FIG. 4A is a drawing to show the whole shape of the microchannel chip 70 and measurement positions set apart in a horizontal direction in a reaction detection cell; FIG. 4B is a sectional view taken on line Y-Y in FIG. 4A; FIG. 4C is a drawing to show the whole shape of the microchannel chip 70 carrying a solidified reagent; and FIG. 4D is a sectional view taken on line Y-Y in FIG. 4C;
FIG. 5 is a sectional view on an enlarged scale with a lid 76 as a bottom face in the sectional view taken on line Y-Y in FIGS. 4A and 4C;
FIG. 6 is a fluorescence strength graph to show a reagent diffusion situation in the cell measured at the measurement positions shown in FIG. 4A;
FIG. 7 is a fluorescence strength graph to show a reagent diffusion situation in a vertical direction in the reaction detection cell shown in FIG. 4A;
FIG. 8A is a schematic representation a cell carrying a reagent in a microchannel chip in a related art; FIG. 8B is a schematic representation of a state in which a reagent is put as a drip on an inner wall face of the cell in FIG. 3A; and FIG. 8C is a schematic representation of action when inspected liquid flows into the cell;
FIG. 9 is a drawing to show the nucleic acid amplification reactions when primers provided by mixing gelatin as the heat soluble binder and drying and solidifying were used;
FIG. 10 is a drawing to show the nucleic acid amplification reactions when primers provided by mixing gelatin and hydroxypropylcellulose (HPC) and drying and solidifying were used; and
FIG. 11 is a drawing to show the nucleic acid amplification reactions when liquid primers provided by mixing gelatin and hydroxypropylcellulose (HPC) were used.

### Detailed Description of the Invention

A preferred embodiment of a microchannel chip according to the invention will be discussed in detail with reference to the accompanying drawings.

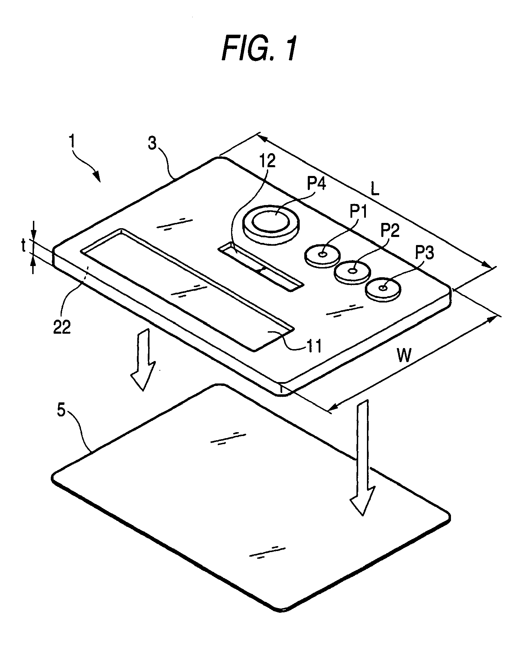

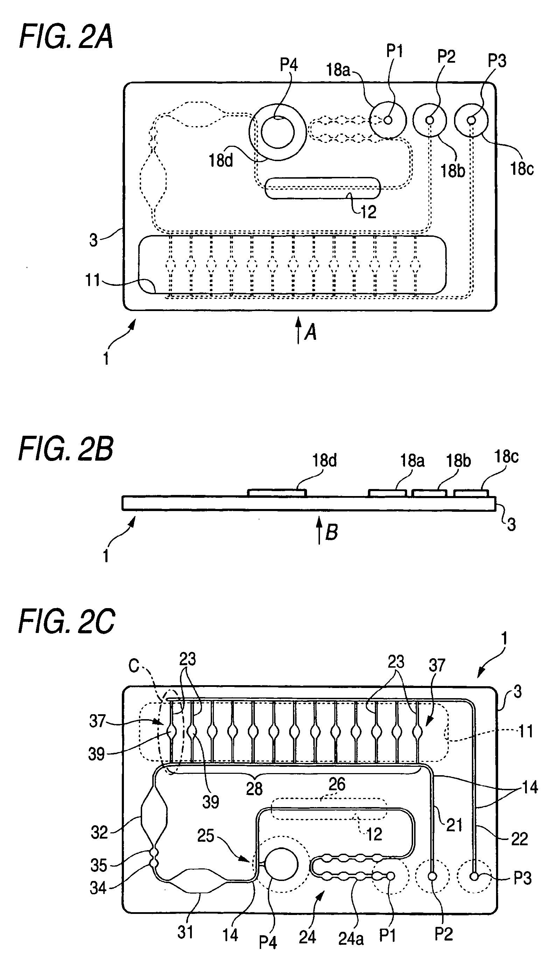

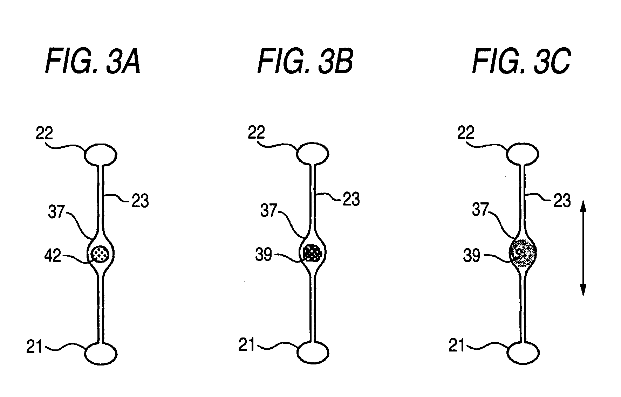

FIG. 1 is an exploded perspective view of one embodiment of a microchannel chip according to the invention, FIGS. 2A to 2C are schematic representations of the configuration of a channel substrate forming the microchannel chip shown in FIG. 1, FIG. 2A is a plan view of the channel substrate, FIG. 2B is a drawing taken on arrow A in FIG. 2A, FIG. 2C is a drawing taken on arrow B in FIG. 2B, FIG. 3A is an enlarged drawing of part C of a micro channel shown in FIG. 2C, FIG. 3B is a schematic representation of a state in which a reagent is put as a drip on an inner wall face of a cell in FIG. 3A, and FIG. 3C is a schematic representation of action when inspected liquid flows into the cell.

A microchannel chip (also simply called "chip") 1 shown in FIGS. 1 and 2A to 2C is set in an inspection apparatus (not shown) for use and is discarded after once used. In the embodiment, blood (whole blood) of a sample is poured into the microchannel chip 1. The microchannel chip 1 is set in the inspection apparatus, whereby the sample liquid is handled by a physical action force from the outside of the chip and, for example, a plurality of target genes of single nucleotide polymorphism are inspected; reaction to amplify the nucleic acid of the target sequence isothermally and specifically and detection thereof can be realized on the chip 1 as shown in JP-A-2005-160387. Accordingly, for example, the target nucleic acid is amplified and is detected, whereby it is made possible to amplify and detect the target nucleic acid specific to the pathogen causing an infectious disease, and it is made possible to determine whether or not the pathogen exists in the sample, etc.

In the embodiment, the physical action force is a pneumatic action force (pneumatic drive force) generated by air supply or air suction from a port part PT provided at the start point and the end point of a liquid channel. Therefore, it is made possible to perform move control of liquid supplied to the liquid channel to any desired position in the liquid channel by air supply or air suction acted on the start point and the end point of the liquid channel. At this time, the liquid is held in a state in which it is clamped in the gas intervening between the start point and the tip part and between the rear end part and the end point and is not broken midway by the action of a tensile force.

The DNA amplification reaction is kept at a temperature at which the activity of the used enzyme can be maintained constant by isothermal amplification reaction. The term "isothermal" mentioned here refers to such an almost constant temperature at which an enzyme and a primer can function substantially. Further, the expression "almost constant temperature" is used to mean that temperature change to such an extent that the substantial function of an enzyme and a primer is not impaired is allowed.

The microchannel chip 1 is manufactured by injection molding of a thermoplastic polymer. Although the polymer to be used is not limited, it is desirable that the polymer should be optically transparent, have high heat resistance, be chemically stable, and be easily injection molded; COP, COC, PMMA, etc., is preferred. The expression "optically transparent" is used to mean that permeability is high in the wavelengths of excitation light and fluorescence used for detection, that scattering is small, and autofluorescence is small. Since the microchannel chip 1 has light-transmitting property for making it possible to detect fluorescence, for example, cybergreen is used for a detection reagent and it is made possible to measure fluorescence emitted as it is intercalated into double stranded DNA amplified by reaction. Accordingly, it is made possible to detect the presence or absence of a gene sequence as a target.

In FIG. 1 and FIG. 2A, a channel substrate 3 has a thickness dimension t, a width dimension W, and a length dimension L set to a portable appropriate size as space saving. The channel substrate 3 is formed on a surface with excavations 11 and 12 of recesses for attaching an external heating unit, a sample inputting port P1 provided communicating with one end of a micro channel 14 described later, control ports P2 and P3 provided communicating with the micro channel 14 for controlling a move of a sample in the micro channel 14, and a reagent inputting port P4 provided communicating with somewhere in the micro channel 14 for inputting a mixed substance such as a reagent for preliminary treatment into the micro channel 14.

The ports P1 to P4 opened to the surface of the channel substrate 3 are surrounded by ring-like swellings 18a, 18b, 18c, and 18d swelling from other portions of the channel substrate 3 (see FIG. 2B), piping is connected to the swellings 18a, 18b, 18c, and 18d through port pads, and a pump for the inspection apparatus to perform liquid delivery drive (sample move operation in the micro channel 14) is connected to each piping through a valve.

The ports P1 to P4 are formed as through holes piercing the channel substrate 3 and communicate with the micro channel 14 formed on the back of the channel substrate 3. The micro channel 14 is formed as grooves in the channel substrate 3 and becomes a channel as the open faces are sealed with a lid 5. The micro channel 14 is made up of a first micro channel groove 21 and a second micro channel groove 22 communicating with the first micro channel groove 21 through a plurality of branch channels 23.

The first micro channel groove 21 has a base end communicating with the sample inputting port P1 and a termination communicating with the control port P2. The second micro channel groove 22 has a base end side communicating with somewhere in the first micro channel groove 21 through a plurality of branch channels 23 and a termination communicating with the control port P3.

A first mixing section 24 is provided at a position to the port P1 of the termination of the first micro channel groove 21 and further a heating section 26 is set in the first micro channel groove 21 between a converging section 25 where the reagent inputting port P4 communicates with the first micro channel groove 21 and the first mixing section 24.

The first mixing section 24 is provided by placing a plurality of mixing cells 24a each shaped like a hexagon formed by widening the groove width of the first micro channel groove 21 in series with a predetermined spacing along the flow direction. An external heating unit (of the inspection apparatus) is placed below the lid 5 providing the bottom of the micro channel 14 and can heat from the bottom of the micro channel 14.

The heating section 26 is a part for raising the temperature of a mixed substance flowing in the micro channel 14 to a predetermined temperature by the heating unit attached to the lid 5 of the bottom side of the excavation 12 position placed on the surface side of the channel substrate 3.

On the first micro channel groove 21, a second mixing section 31 and a third mixing section 32 are disposed in order from the side of the converging section 25 between the converging section 25 and a dispensing section 28 with a plurality of branch channels 23 connected to the first micro channel groove 21. Further, two reagent carrying sections 34 and 35 holding a dry reagent to be brought into contact with and reacted with a sample as another preliminary treatment are provided between the second mixing section 31 and the third mixing section 32.

Each of the reagent carrying sections 34 and 35 is formed with a channel space having a larger channel cross section than the micro channel 14 by widening the groove width of the first micro channel groove 21 like the mixing cell 24a in the first mixing section 24, and a dry reagent is held at a position of the top face of the channel space.

In the microchannel chip 1 of the embodiment, the sample as the liquid reagent introduced into the micro channel 14 from the port P1 is made to go and return between the two mixing sections 31 and 32 of dissolving and mixing cells, thereby uniformly dissolving and mixing the dry reagent carried on the reagent carrying sections 34 and 35 in a dry state.

As the optimum portion that can incorporate the invention, there are a plurality of branch channels 23 forming the dispensing section 28. The branch channels 23 are connected in parallel with the first micro channel groove 21 and the second micro channel groove 22, and each of the branch channels 23 has a reaction detection cell 37 with a channel widened like a hexagon at a midpoint. The reaction detection cell 37 carries a primer as a solidified reagent 39 for detection on the channel wall face on the upper face side at the top position in the gravity direction in the reaction detection cell 37 and when a sample already subjected to preliminary treatment flows into the reaction detection cell 37, the sample is analyzed by the reaction of the sample and the reagent with each other.

Solidified reagents 39 of primers different in components are carried on the reaction detection cells 37 of the branch channels 23, whereby multiple types of analysis on the sample can be conducted at a time.

The dispensing section 28 can be heated by the heating unit of the inspection apparatus abutting the lid 5 of the bottom side of the excavation 11 position formed on the surface side of the channel substrate 3; it is heated to a temperature appropriate for reaction promotion of the solidified reagent 39 for analysis carried on the reaction detection cell 37.

In the embodiment, the solidified reagent 39 is carried on the reaction detection cell 37 of each branch channel 23 as follows:

First, a proper amount of liquid or gel reagent 42 mixed with a heat soluble binder is put as a drip on the channel wall face on the upper face side of the reaction detection cell 37, as shown in FIG. 3A. The moisture of the reagent 42 is evaporated, whereby a state in which the reagent 39 in a dry state is fixedly secured to the inner wall face of the cell is obtained as shown in FIG. 3B.

In the embodiment, the reagent to be carried on the reaction detection cell 37 is a primer of an origin for causing nucleic acid amplification reaction to occur in blood of a sample.

Gelatin or hydroxypropylcellulose (HPC) whose dissolution promotion temperature is 35°C or more and 60°C or less is selected as the heat soluble binder mixed with the reagent 42.

In the reagent 39 in the solidification state, the content of gelatin or hydroxypropylcellulose (HPC) of a heat soluble binder is set in the range of 20% to 98% in terms of weight ratio considering the dissolution speed and the reaction speed into blood of a sample.

The heating unit provided in the dispensing section 28 keeps the inside of each reaction detection cell 37 constantly at a stipulated temperature at which reaction of the solidified reagent 39 becomes active for realizing isothermal reaction with the solidified reagent 39.

With the described microchannel chip 1, opening/closing a valve of inspection apparatus piping connected to the control ports P2 and P3 or pressurization or suction of a pump connected to the valve is controlled appropriately, whereby the position of the sample input to the micro channel 14 from the sample inputting port P1 can be moved to any desired position of the micro channel 14 and the sample already subjected to mixing treatment for preliminary treatment in the mixing sections 24, 31, 32, etc., is dispensed into the reaction detection cells 37, whereby multiple types of analysis on the sample are conducted.

According to the microchannel chip 1 described above, a substance having adhesion is adopted as a heat soluble binder previously mixed with the reagent 39 carried on the reaction detection cell 37 as a predetermined position in the micro channel 14, whereby the adhesive strength between the reagent and the channel wall face in the reaction detection cell 37 can improved drastically.

Thus, the reagent is prevented from peeling off from the carry position and flowing as it only comes in contact with the inspected liquid (sample) supplied in the micro channel 14.

Moreover, when the temperature of the inspected liquid is not raised to a stipulated temperature of the dissolution promotion temperature of a heat soluble binder, dissolution of the heat soluble binder is not promoted, and therefore dissolution of the reagent 39 is not promoted either.

In other words, if the heating unit provided in the dispensing section 28 raises the temperature of the inspected liquid introduced into the carry position of the reagent 39 to the stipulated temperature, dissolution of the heat soluble binder coming in contact with the inspected liquid is promoted and at the same time, dissolution and mixing of the reagent 39 are also promoted as shown in FIG. 3C and it is ensured that dissolving treatment and mixing treatment are started and continued at the initial carry position. Thus, for example, the inspected liquid is retained for a given time in a given section with the carry position of the reagent 39 between (for example, in the range of the branch channel 23) or the inspected liquid is made to go and return in a given section with the carry position of the reagent 39 between, whereby the reagent can be reliably dissolved and mixed in the inspected liquid and accuracy of the dissolving treatment and the mixing treatment can be improved.

Further, if the characteristic of heat soluble binder is previously adjusted so that the dissolution promotion temperature of the heat soluble binder becomes the reaction promotion temperature of the reagent 39 and the inspected liquid, the dissolved and mixed reagent 39 can be made to react with the inspected liquid efficiently, and the later reaction treatment and analysis treatment can be executed on the periphery of the initial carry position.

That is, in addition to the dissolving treatment and the mixing treatment, the later reaction treatment and analysis treatment can be executed only at the initial carry position of the reagent 39 or on the periphery of the initial carry position.

Therefore, the number of the treatment positions on the micro channel can be reduced as compared with the related art case where the dissolving treatment, the mixing treatment, the reaction treatment, and the analysis treatment can be executed at different positions on the micro channel, so that simplifying of the micro channel 14 and the branch channels 23 formed on the chip and shortening of the channel length can also be realized for miniaturizing the chip and reducing the manufacturing cost.

In the embodiment, a substance whose dissolution promotion temperature becomes 35°C or more and 60°C or less is selected as the heat soluble binder mixed with the solidified reagent 39. In so doing, the dissolution promotion temperature of the solidified reagent 39 can be shifted from the normal temperature for precisely managing the dissolution start timing and at the same time, for example, if the reagent 39 is a dry primer used for the purpose of amplification and detection of a target nucleic acid in blood, the reaction temperature of the dry primer can also be maintained for promoting the reaction.

In the embodiment, gelatin or hydroxypropylcellulose (HPC) is adopted as the heat soluble binder.

In so doing, gelatin or hydroxypropylcellulose (HPC) has heat solubility capable of being set to the condition of the above-mentioned dissolution promotion temperature ranging from 35°C to 60°C and at the same time, is also has strong adhesion, so that carrying of the reagent 39 to a predetermined position of the reaction detection cell 37 on the branch channel 23 is facilitated and uniform mixing and reaction promotion can be realized by appropriate management of the dissolution temperature and the reaction temperature described above for advancing treatment efficiently,

In the embodiment, the content of the heat soluble binder is regulated so that it becomes in the range of 20% to 98% at the weight ratio of the heat soluble binder to the reagent in the solidification state.

The heat soluble binder contained in the reagent 39 carried on the channel becomes an impurity when it dissolves into inspected liquid. Therefore, it is desirable that the content of the heat soluble binder should be lowered as much as possible. However, to optimally adjust the required time and the reaction speed for uniformly dissolving and mixing the reagent 39 in inspected liquid, it might often be effective to suppress the dissolution speed of the reagent 39 by raising the content of the heat soluble binder. From such a viewpoint, if the content of the heat soluble binder is regulated as mentioned above, solubility of the reagent 39 can be controlled flexibly in response to the composition, the analysis purpose, etc., of the inspected liquid.

In the embodiment, the reagent 39 is a reagent for causing nucleic acid amplification reaction to occur; in so doing, the nucleic acid amplification reaction can be executed efficiently at the carry position of the reagent 39 or on the periphery of the carry position.

Further, in the embodiment, the heating unit provided in the dispensing section 28 keeps the inside of each reaction detection cell 37 at a constant temperature, so that the nucleic acid amplification reaction of inspected liquid with the solidified reagent 39 becomes isothermal amplification reaction and the activity of a used enzyme can be maintained constant and thus the nucleic acid amplification reaction stably proceeds, the reliability and the treatment accuracy of the reaction treatment can be improved, and the required time can be shortened.

Further, in the embodiment, the reagent 39 is a primer of the origin of the nucleic acid amplification reaction of inspected liquid and thus the dissolution start of the reagent 39 at the carry position of the reagent 39 can be controlled so that it is the start of the nucleic acid amplification reaction, and management of the reaction time, etc., is facilitated, whereby the nucleic acid amplification reaction can be well controlled and advanced.

The inventor of the invention conducted an experiment to check the dissolution and mixing characteristics of the reagent in the above-described embodiment of using gelatin as a heat soluble binder and carrying the solidified reagent 39 mixed with the heat soluble binder on the reaction detection cell 37.

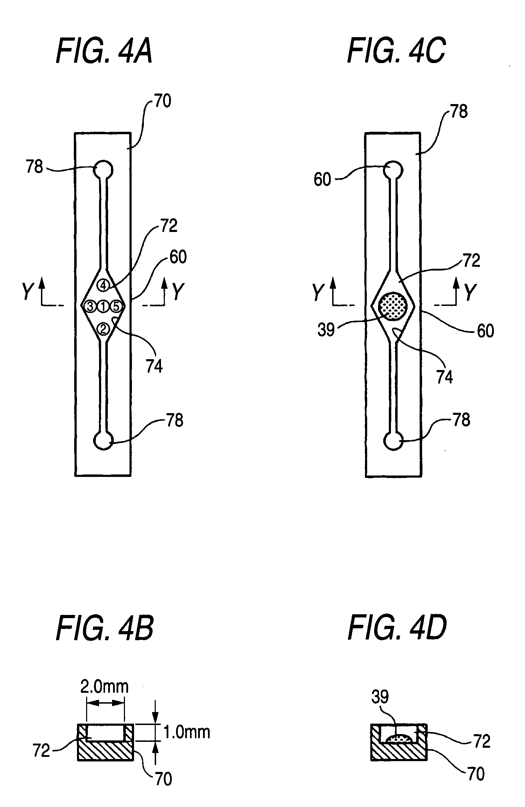

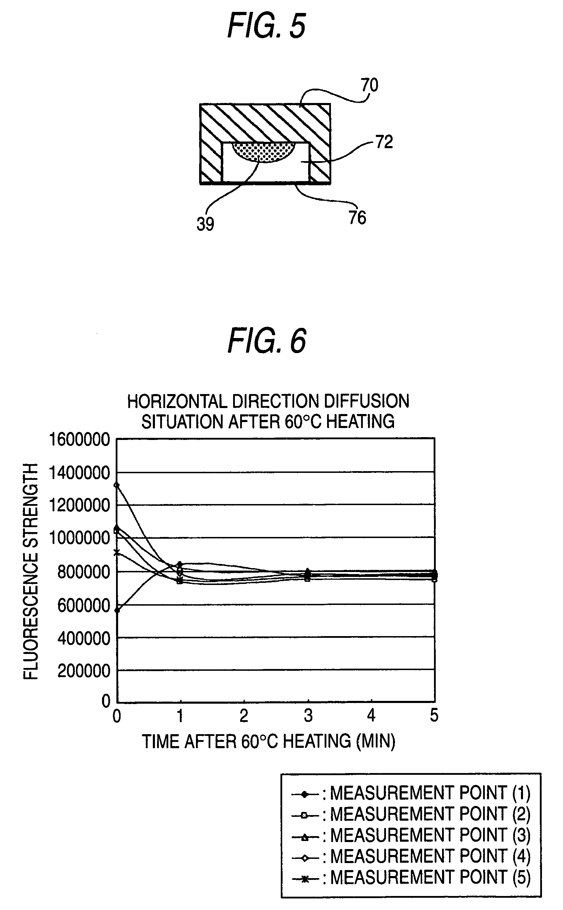

FIGS. 4A, 4B, 4C, and 4D and FIG. 5 show the structure of a microchannel chip 70 used for the experiment. The structure of the microchannel chip 70 used for the experiment imitates the structure of the reaction detection cell 37 shown in FIG. 2C. FIG. 4A is a drawing to show the whole shape of the microchannel chip 70 and measurement positions set apart in a horizontal direction in a reaction detection cell, FIG. 4B is a sectional view taken on line Y-Y in FIG. 4A, FIG. 4C is a drawing to show the whole shape of the microchannel chip 70 carrying a solidified reagent, and FIG. 4D is a sectional view taken on line Y-Y in FIG. 4C. FIG. 5 is a sectional view taken on line Y-Y on an enlarged scale.

A reaction detection cell 72 is placed at the center of the microchannel chip 70 and through liquid delivery ports 78 placed at both end positions are communicated with the reaction detection cell. At least the position of the reaction detection cell 72 of a side 60 of the microchannel chip 70 is mirror-finished and the inside of the reaction detection cell 72 can be detected. Further, the opening side of the reaction detection cell 72 is closed by a lid 76 which becomes a bottom face. The dimensions of the reaction detection cell 72 are set to 2.0 mm in width and 1.0 mm in depth. The dimensions imitate those of the actual reaction detection cell, but can be changed in design as required and the dimensions are not limited to them.

Next, a procedure of carrying a solidified reagent 39 on the reaction detection cell 72 is shown.

A liquid synthetic polymer (manufactured by OPERON) was used as the solidified reagent 39 to be carried on the reaction detection cell 72 and was mixed with a solution, 5% at a volume ratio, of dissolving PanomerTM9 random oligodeoxynucleotide, Alexa FluorR 488 Conjugate (manufactured by Invitrogen) of a fluorescence-labeled primer to quantify the dissolution and mixing characteristics with sterile water so that it becomes 100 µM, and a primer solution was prepared.

Further, the primer solution and a gelatin solution (provided by dissolving beef bone gelatin (manufactured by Nitta Gelatin) with a 10-mM Tris buffer (provided by dissolving Tris hydrochloric acid, ph 7.5 (1 M) (manufactured by Invitrogen) with sterile water as a 100-fold dilution factor) so that it becomes 2.5% (Weight/Volume) were mixed at a volume ratio of 4:1 and a liquid primer solution containing gelatin of a heat soluble binder was prepared. Since the gelatin solution is solidified at the normal temperature, it was heated to 45°C and was made to gel and then was mixed with the primer solution. The post-mixed solution has a gelatin concentration of 0.5% (Wei.ght/Volume) and thus is not solidified at the normal temperature.

Subsequently, 1.0 µL of the liquid primer solution in which gelatin of a heat soluble binder was mixed was put as a drip on an inner wall face 74 of the reaction detection cell with a pipette and then was dried for two hours in a desiccator containing silica gel, whereby the liquid synthetic polymer of the solidified reagent 39 was carried on the reaction detection cell 72. (See FIGS. 4C and 4D.)

Next, an experiment method to check the dissolution and mixing characteristics of the solidified reagent is shown.

The microchannel chip 70 with the solidified reagent 39 carried thereon was covered with seal-TSRT2 (manufactured by Excel Scientific) of an adhesive seal as the lid 76. With the side of the lid 76 down, 0.2% Tween20 water solution (provided by diluting 10% Tween20 (manufactured by Wakou Jyunyaku) with sterile water at a 50-fold dilution factor) was delivered with a pipette from a liquid delivery port 78 and was filled into the reaction detection cell 72 and then the reaction detection cell 72 (microchannel chip 70) was heated to 60°C of the reaction promotion temperature of the primer with a plate heater (not shown) from the bottom face (the side of the lid 76) (see FIG. 5) and a fluorescence brightness distribution in the horizontal direction and the gravity direction was measured. In the horizontal direction, LAS-3000 (manufactured by FUJIFILM Corporation) was used to conduct measurement from the upper face side of the reaction detection cell 72 and in the gravity direction, a stereo fluorescence microscope system VB-G25 (manufactured by KEYENCE) was used to conduct measurement from the side of the mirror-finished detection cell side 60.

In FIG. 4A, five brightness measurement points set apart in the horizontal direction in the reaction detection cell are indicated by digits.

FIG. 6 shows change in brightness measured at each measurement point shown in FIG. 4A when treatment in the reaction detection cell is executed while the temperature of inspected liquid introduced into the reaction detection cell is maintained at 60°C.

When the 60°C isothermal heating state was maintained, the fluorescence strengths converged on roughly the same fluorescence strength about for three minutes anywhere in the horizontal direction. Accordingly, it can be checked that the reagent 39 was dissolved and mixed roughly uniformly in the horizontal direction in the channel.

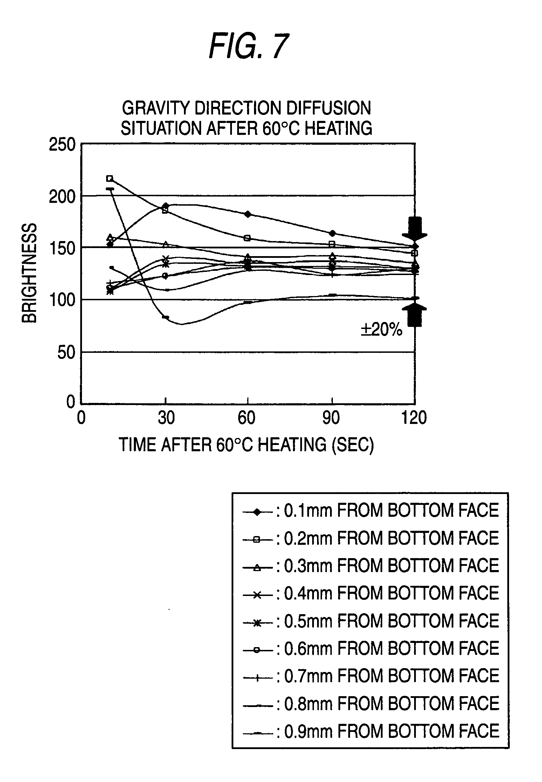

FIG. 7 shows measurement of a brightness distribution in the gravity direction at points by changing the distance from the bottom face in the reaction detection cell (cell depth 1.0 mm); it can be checked that brightness distribution variations at the points in the reaction detection cell decrease with the passage of the heating time, that brightness variations converge within ±20% after the expiration of 120 seconds, that the component of the solidified reagent (primer) is diffused roughly uniformly over roughly all area in the vertical direction in the reaction detection cell, and that the solidified reagent (primer) is dissolved and mixed uniformly in the inspected liquid.

Next, using gelatin and hydroxypropylcellulose (HPC) as heat soluble binders, the inventor of the invention conducted an experiment to check the reactivity of the reagent in the above-described embodiment of carrying the solidified reagent 39 with which the heat soluble binder is mixed on the reaction detection cell 37.

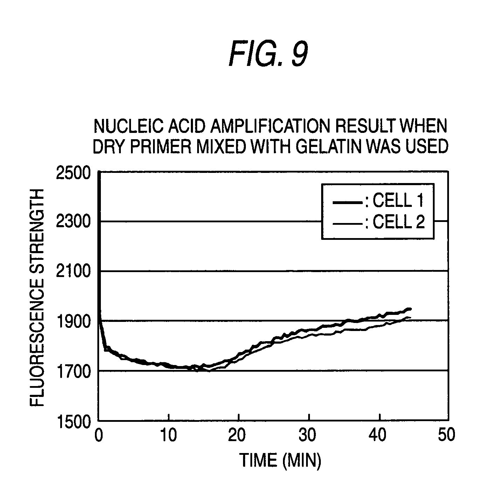

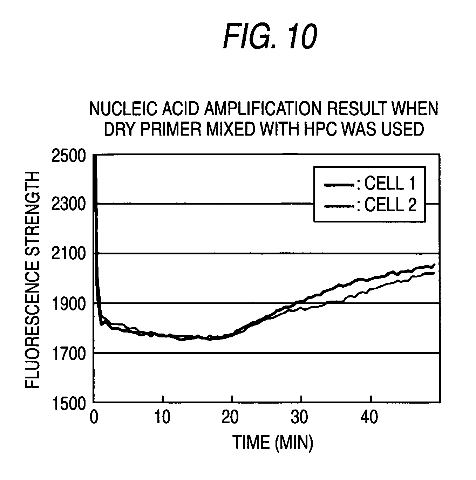

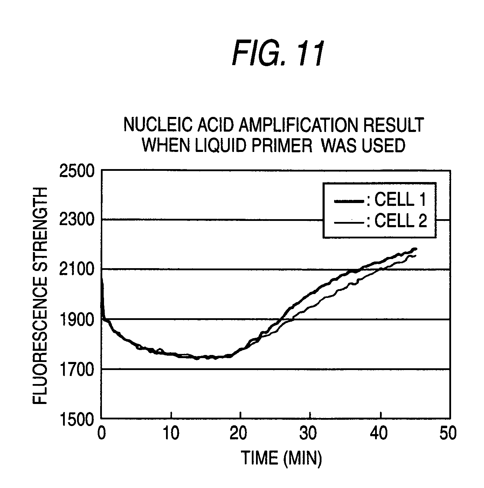

FIG. 9 shows nucleic acid amplification reactions when primers provided by mixing gelatin as heat soluble binders and drying and solidifying were used, FIG. 10 shows nucleic acid amplification reactions when primers provided by mixing gelatin and hydroxypropylcellulose (HPC) as heat soluble binders and drying and solidifying were used, and FIG. 11 shows nucleic acid amplification reactions when liquid primers provided by mixing gelatin and hydroxypropylcellulose (HPC) were used, respectively. A liquid synthetic polymer (manufactured by OPERON) was used as the solidified reagent 39 to be carried on the reaction detection cell 72, each of a gelatin solution [provided by dissolving beef bone gelatin (manufactured by Nitta Gelatin) with a 10-mM Tris buffer (provided by dissolving Tris hydrochloric acid, ph 7.5 (1 M) (manufactured by Invitrogen) with sterile water as a 100-fold dilution factor) so that it becomes 2.5% (Weight/Volume)] and a hydroxypropylcellulose solution [provided by dissolving hydroxypropylcellulose (manufactured by Nihon Sotatsu) with a 10-mM Tris buffer {provided by dissolving Tris hydrochloric acid, ph 7.5 (1 M) (manufactured by Invitrogen) with sterile water as a 100-fold dilution factor} so that it becomes 2.5% (Weight/Volume) ] was mixed into the primer solution at a volume ratio of 4:1 to prepare liquid primer solutions containing gelatin and hydroxypropylcellulose (HPC) as heat soluble binders, and each of the liquid primer solutions was put as a drip on the inner wall face 74 of the reaction detection cell 72 with a pipette and then was dried for two hours in a desiccator containing silica gel, whereby each of the synthetic polymers of the solidified reagent 39 was carried on the reaction detection cell 72.

To use gelatin and hydroxypropylcellulose (HPC) as heat soluble binders, rising of fluorescence strength was recognized in about 18 min after the reaction start as with the case of using the liquid primer and it was seen that the nucleic acid amplification reactions proceeded like the nucleic acid amplification reaction when the liquid primer was used.

The reagent carrying structure wherein a reagent mixed with a heat soluble binder such as gelatin or hydroxypropylcellulose (HPC) is previously carried and it is made possible to control the dissolution start point of the reagent by adjusting the temperature of the inspected liquid brought into contact with the reagent may be applied not only to the reaction detection cell 37 in the dispensing section 28, but also to each mixing cell 24a in the mixing section 24 and the reagent carrying sections 34 and 35. Accordingly, the scale of the two mixing sections 31 and 32 positioned upstream from the dispensing section 28 and the heating section 26 can be reduced or a part can be decreased for further shortening the overall strength of the micro channel 14 and miniaturizing the chip.

A technique of mixing a heat soluble binder such as gelatin or hydroxypropylcellulose (HPC) and carrying the mixture on the microchannel chip may be applied not only to the primer of the embodiment, but also to an enzyme of a reagent required for nucleic acid amplification reaction and detection, a substrate of dNTP mix, etc., cybergreen of an inspection reagent, etc.

As kinds of the heat soluble binder, in addition to above-mentioned gelatin or hydroxypropylcellulose (HPC), collagen and agarose are exemplified.

In the microchannel chip according to the invention, a substance having adhesion is adopted as a heat soluble binder to be mixed with the reagent carried at a predetermined position in the micro channel, whereby the adhesive strength between the reagent and the channel wall face can improved drastically. Thus, the reagent is prevented from peeling off from the carry position and flowing as it only comes in contact with the inspected liquid supplied in the micro channel. Moreover, when the temperature of the inspected liquid is not raised to a stipulated temperature, dissolution of the heat soluble binder is not promoted, and therefore dissolution of the reagent is not started either. In other words, if the temperature of the inspected liquid introduced into the carry position of the reagent is raised to the stipulated temperature, dissolution promotion of the heat soluble binder coming in contact with the inspected liquid starts and dissolution and mixing of the reagent start and it is ensured that dissolving treatment and mixing treatment are started and continued at the initial carry position. Thus, for example, the inspected liquid is retained for a given time in a given section with the carry position of the reagent between or the inspected liquid is made to go and return in a given section with the carry position of the reagent between, whereby the reagent can be reliably dissolved and mixed in the inspected liquid and accuracy of the dissolving treatment and the mixing treatment can be improved.

Further, if the characteristic of a heat soluble binder is previously adjusted so that the dissolution promotion temperature of the heat soluble binder becomes the reaction promotion temperature of the reagent and the inspected liquid, the dissolved and mixed reagent can be made to react with the inspected liquid efficiently, and the later reaction treatment and analysis treatment can be executed on the periphery of the initial carry position. That is, in addition to the dissolving treatment, the mixing treatment, and the amplification treatment, the later reaction treatment and analysis treatment can be executed only at the initial carry position of the reagent or on the periphery of the initial carry position. Therefore, the number of the treatment positions on the micro channel can be reduced as compared with the related art case where the dissolving treatment, the mixing treatment, the reaction treatment, and the analysis treatment can be executed at different positions on the micro channel, so that simplifying of the micro channel formed on the chip and shortening of the channel length can also be realized for miniaturizing the chip and reducing the manufacturing cost.

The entire disclosure of each and every foreign patent application from which the benefit of foreign priority has been claimed in the present application is incorporated herein by reference, as if fully set forth.

## Claims

1. A microchannel chip for introducing an inspected liquid, comprising:
a micro channel; and
a reagent that is mixed with a heat soluble binder and is carried at a predetermined position in the micro channel,
wherein dissolution of the reagent is promoted at the predetermined position as temperature of the inspected liquid rises from temperature when the inspected liquid is introduced.

2. The microchannel chip according to claim 1,
wherein the temperature of the inspected liquid rises to 35°C or more and 60°C or less.

3. The microchannel chip according to claim 1,
wherein the heat soluble binder is gelatin or hydroxypropylcellulose.

4. The microchannel chip according to claim 1,
wherein the heat soluble binder is contained in the reagent in a solidified state 20% or more and 98% or less at a weight ratio of the heat soluble binder to the reagent.

5. The microchannel chip according to claim 1,
wherein the reagent causes nucleic acid amplification reaction.

6. The microchannel chip according to claim 5,
wherein the nucleic acid amplification reaction is executed at a constant temperature.

7. The microchannel chip according to claim 1,
wherein the reagent is a primer of an origin of nucleic acid amplification reaction.
